# EUROPÄISCHE PATENTSCHRIFT

(11) **EP 4 431 176 B1**
(45) Veröffentlichungstag und Bekanntmachung des Hinweises auf die Patenterteilung: **19.08.2026**
(21) Anmeldenummer: 24184414.1
(22) Anmeldetag: 23.06.2020
(51) Int. Cl.: A61K 9/16, A61J 3/00, A61K 31/00, A61K 36/00, B01J 2/00, A61J 3/02, A61K 31/05, A61K 31/352, A61K 36/185, B01J 2/16

(54) **VORRICHTUNG ZUR HERSTELLUNG EINES IM WESENTLICHEN IM WÄSSRIGEN MILIEU LÖSBAREN CANNABINOID-GRANULATS**
APPARATUS FOR PRODUCTION OF A GRANULAR CANNABINOID MATERIAL ESSENTIALLY SOLUBLE IN AQUEOUS MEDIUM
DISPOSITIF DE FABRICATION DE GRANULÉS DE CANNABINOÏDE SOLUBLES ESSENTIELLEMENT DANS UN MILIEU AQUEUX

(30) Priorität: 26.07.2019 DE 102019211195
(43) Veröffentlichungstag der Anmeldung: 18.09.2024
(62) Dokumentnummer(n) der früheren Anmeldung(en) nach Art. 76 EPÜ: 20734375.7 / 4 003 307
(73) Patentinhaber: ADD Advanced Drug Delivery Technologies, Ltd., 4133 Pratteln (CH)
(72) Erfinder: Jacob, Michael, 99427 Weimar (DE); Nowak, Mirko, 79540 Lörrach (DE)
(74) Vertreter: Patentanwälte Magenbauer & Kollegen Partnerschaft mbB

(56) Entgegenhaltungen:
- DE-A1- 10 326 231
- DE-A1- 102016 121 050
- US-A1- 2019 022 009

## Beschreibung

Die Erfindung betrifft eine Vorrichtung zur Herstellung eines im Wesentlichen im wässrigen Milieu lösbaren Cannabinoid-Granulats, wobei eine Matrixflüssigkeit aus einer ein Cannabinoid lösenden ersten Flüssigkeit oder aus einer ein Cannabinoid lösenden ersten Flüssigkeit und einer mit der ersten Flüssigkeit eine Emulsion bildenden zweiten Flüssigkeit und einem in der ersten Flüssigkeit oder der Emulsion gelösten Cannabinoid hergestellt wird, wobei die Matrixflüssigkeit in einem Fluidisierungsapparat (18) durch eine Sprühgranulation, Sprühagglomeration oder Sprühverkapselung konvektiv zu einem Cannabinoid-Granulat getrocknet wird.

Vorrichtungen zur Herstellung von insbesondere oralen Darreichungsformen eines Cannabinoids gehören seit langem zum Stand der Technik.

WO 02/064109 A2 offenbart pharmazeutische Formulierungen zur Verwendung bei der Verabreichung von lipophilen Arzneimitteln über Schleimhautoberflächen. Insbesondere werden pharmazeutische Formulierungen zur Verwendung bei der Verabreichung eines lipophilen Arzneimittels über eine Schleimhautoberfläche bereitgestellt, die bei Hydratisierung eine Emulsion bilden, die das lipophile Arzneimittel enthält, insbesondere Arzneimittel, die als Wirkstoffe bestimmte Kombinationen von Cannabinoiden in vordefinierten Verhältnissen enthalten, wobei das Arzneimittel in der Lage ist, an einer Schleimhautoberfläche zu haften und eine kontrollierte Freisetzung des Medikaments zu ermöglichen.

In der US 2016/0143972 A1 wird ein Verfahren zur Herstellung einer festen Darreichungsform eines Cannabinoids offenbart, wobei die feste Darreichungsform des Cannabinoids im Wesentlichen in einer wässrigen Lösung löslich ist. Das Verfahren umfasst das Lösen eines Cannabinoids und eines oder mehrerer Emulgatoren in einem oder mehrerer Lösungsmittel, um eine oder mehrere kombinierte Lösungen zu erhalten und ferner ein Trocknen des einen oder der mehreren Lösungsmittel von dem einen oder den mehreren Lösungsmitteln umfassenden Kombinationslösungen, um die feste Darreichungsform des Cannabinoids zu erhalten.

Nachteilig ist, dass die im Stand der Technik genannten festen Darreichungsformen eines Cannabinoids eine unzureichende systemische Absorption aufweisen und daher die Bioverfügbarkeit der Cannabinoide gering ist, da die Korngrößenverteilungen der festen Darreichungsformen nicht sehr homogen sind und zudem eine schlechte Rieselfähigkeit der oral verabreichten ggf. festen Darreichungsformen besteht.

US 2019/022009 A1 offenbart ein Verfahren zur Herstellung eines biopolymerischen hämostatischen Pulvers, das das Hinzufügen einer Mischung aus einer organischen Säure, einer Kombination von Alkoholen und einem Bioadhesiv in einen Reaktor umfasst, um eine Bindemittellösung zu erhalten, und einen Schritt zur Einbringung auf einer Basis des unteren Teils eines Wirbelschichtreaktors eines Polysaccharids, das durch einen Luftstrom, der bei kontrollierter Temperatur und Geschwindigkeit injiziert wird, angetrieben wird; wobei auf dieses Fluid und Mikropartikel des Polysaccharids von der Oberseite des genannten Wirbelschichtreaktors die Bindemittellösung gesprüht wird und wobei das Polysaccharid Chitosan ist, die organische Säure Essigsäure ist, die Alkoholkombination aus Alkohol und Polyalkohol besteht und das Bioadhesiv Polyvinylalkohol ist. Das Produkt enthält zwischen 55 % und 85 % w/w eines Polysaccharids; zwischen 10 % und 40 % w/w einer organischen Säure; bis zu 17 % w/w einer Kombination von Alkoholen und bis zu 3 % w/w eines Bioadhesivs; wobei die Alkoholkombination aus 95 % Alkohol und 5 % Polyalkohol besteht und die Bindeflüssigkeit in einer Menge von zwischen 50 % und 150 % p/p auf das Polymer gesprüht wird.

DE 103 26 231 A1 betrifft ein Verfahren zur Herstellung von Enzym-Granulaten. Aufgabe der Erfindung ist es, ein Verfahren zur Herstellung von Enzym-Granulaten zu schaffen, bei dem die Enzym-Granulate kontinuierlich oder chargenweise unter weitester Vermeidung von Temperaturungleichverteilungen im Herstellungsprozess und bei Erhöhung der Ausbeute an Aktivität von Enzymen hergestellt werden können. Gleichzeitig soll die Kontrollierbarkeit der Granulation bei der Herstellung verbessert werden. Erfindungsgemäß erfolgt die Herstellung von Enzym-Granulaten durch eine Verknüpfung zwischen den thermischen Bedingungen in der Sprühzone und den Temperaturbedingungen im übrigen Bereich der Wirbelschicht. Im erfindungsgemäßen Prozess wird dies dadurch erreicht, dass die Zuführung des erhitzten Prozessgases zur Trocknung ausschließlich im Bedüsungsbereich erfolgt. Die sichere Zuführung von Teilchen in den Bedüsungsbereich hinein erfolgt durch die spezielle geometrische Gestaltung des Apparates unter Nutzung der Schwerkraft.

DE 10 2016 121050 A1 geht aus von einem Erzeugnis in Tablettenform bzw. einem Erzeugnis in Kapselform, wobei das Erzeugnis jeweils aus einer getrockneten Darreichungsform einer GcMAF-enthaltenden Formulierung besteht.

Aufgabe der Erfindung ist es daher die Anwendungsbereiche für Verbraucher zu erweitern und die systemische Absorption der Cannabinoide zu erhöhen, sodass sich die Bioverfügbarkeit der Cannabinoide verbessert und gleichzeitig die Rieselfähigkeit der festen Darreichungsform der Cannabinoide zu verbessern, indem eine verbesserte Vorrichtung zur Durchführung eines Verfahrens zur Herstellung von Cannabinoid-Granulaten zur Verfügung gestellt wird.

Diese Aufgabe wird bei einer Vorrichtung eingangs genannter Art dadurch gelöst, dass die Vorrichtung über ein mehrere untereinander über Rohrleitungen zumindest teilweise fluidisch verbundene Behälter aufweisendes Behältersystem zur Herstellung der Matrixflüssigkeit verfügt, das einen Cannabinoid-Einlass, einen Flüssigkeit-Einlass für eine erste Flüssigkeit aus der Gruppe der Lipide, Alkohole, Öle und/oder einer beliebigen Mischung daraus, einen Flüssigkeit-Einlass für eine zweite Flüssigkeit und einen Matrixflüssigkeitsauslass aufweist, wobei die Vorrichtung einen ersten Behälter für die erste Flüssigkeit, einen zweiten Behälter für die zweite Flüssigkeit und einen einen Einlass aufweisenden Behälter für die Emulsion aufweist, und wobei die Vorrichtung ferner einen mit dem Matrixflüssigkeitsauslass des Behältersystems fluidisch verbundenen konvektiven als Fluidisierungsapparat ausgebildeten Trocknungsapparat aufweist. Bevorzugt ist der konvektive Trocknungsapparat als Sprühtrocknungseinrichtung, als Trommel-, als Vakuumtrockner oder als Fluidisierungsapparat ausgebildet. Überraschenderweise wurde herausgefunden, dass sich bei einer konvektiven Trocknung der das Cannabinoid enthaltenden Matrixflüssigkeit in einem Fluidisierungsapparat, insbesondere einem Strahl- oder Wirbelschichtapparat, durch eine Sprühgranulation, Sprühagglomeration oder Sprühverkapselung nach erneuter Auflösung des Cannabinoid-Granulats im wässrigen Milieu eine stabile Emulsion aus erster und zweiter Flüssigkeit bildet, wobei eine Resorbierbarkeit der Cannabinoide deutlich verbessert ist und damit eine optimalere Bioverfügbarkeit gewährleistet wird. Die verbesserte Resorbierbarkeit und optimalere Bioverfügbarkeit der Cannabinoide resultiert aus homogeneren und kleineren Emulsionspartikeln (Öl-Tröpfchen in Wasser), die bspw. durch Aufsprühen und Trocknen, insbesondere Filmtrocknung, der Matrixflüssigkeit auf Trägerpartikel erhalten werden, nach dem erneuten Auflösen der Cannabinoid-Granulate im wässrigen Milieu im Vergleich zu den in der Matrixflüssigkeit enthaltenen Emulsionspartikeln (Öl-Tröpfchen in Wasser) vor der konvektiven Trocknung, vorzugsweise im Fluidisierungsapparat. Zudem wurde überraschend eine Reduzierung des mittleren Durchmessers x sowie der Äquivalenzdurchmesser nach der konvektiven Trocknung im Fluidisierungsapparat festgestellt. Die Cannabinoid-Granulate können sehr gut in Stickpacks abgefüllt und direkt in die Mundhöhle appliziert oder in ein Glas Flüssigkeit gegeben werden bzw. zu Tabletten verpresst werden oder in Kapseln weiterverarbeitet werden.

Nach einer vorteilhaften Ausgestaltung der Vorrichtung ist der Fluidisierungsapparat als Strahlschichtapparat oder als Wirbelschichtapparat ausgebildet. Im Vergleich zu Wirbelschichtapparaten erlauben Strahlschichtapparate beispielsweise die Verdüsung bei sehr geringen Füllmenge und darüber hinaus unterstützen auf der einen Seite die hohen Scherkräfte im Bedüsungsbereich eine gleichmäßige Flüssigkeitsfilmbildung und minimieren auf der anderen Seite Tendenzen zur Agglomeration.

Ganz besonders bevorzug entspricht der Behälter für die Emulsion dem ersten oder zweiten Behälter. Hierdurch wird eine einfache Herstellung der Matrixflüssigkeit bei gleichzeitig getrennt zu verarbeitenden Ausgangsstoffen gewährleistet.

Entsprechend einer zusätzlichen vorteilhaften Weiterbildung der bevorzugten Vorrichtung weist der konvektive Trocknungsapparat, vorzugsweise der Fluidisierungsapparat, eine Düse zur Verdüsung der Emulsion oder Lösung auf. Bevorzugt ist die Düse, bevorzugt eine Zweistoffdüse, konfiguriert, um Tröpfchen mit einer Tröpfchengröße von 1 *µ*m bis 200 *µ*m, bevorzugt von 10 *µ*m bis 100 *µ*m, besonders bevorzugt zwischen 20*µ*m und 60 *µ*m zu versprühen. Ganz besonders bevorzugt haben die versprühten Tröpfchen eine Tröpfchengröße von 25*µ*m bis 40*µ*m, am meisten bevorzugt von 30*µ*m. Durch die Einstellung der Tröpfchengröße mittels der Düse und der die Düse beaufschlagten Druckluft sind die beim Versprühen auftretenden Scherkräfte genau einstellbar, sodass eine sehr homogene Tröpfchengröße der zu versprühenden Emulsion oder Lösung erziel- bzw. erreichbar ist. Die Tröpfchen lagern sich an den Trägerpartikeln, wie Cellulose, Lactose oder Xylitol, an und es findet bevorzugt eine Filmverdampfung statt. Durch die Filmverdampfung erfolgt auf den Trägerpartikeln eine sehr gleichmäßige und Verdampfung der Emulsion oder der Lösung.

Gemäß einer vorteilhaften Weiterbildung der Vorrichtung ist zwischen dem Behältersystem und dem Trocknungsapparat, vorzugsweise dem Fluidisierungsapparat, ein Homogenisator zum Homogenisieren der Matrixflüssigkeit angeordnet. Durch die Homogenisierung wird der mittlere Durchmesser der in der Emulsion vorhandenen ersten Flüssigkeit stark reduziert, sodass das Gemisch aus erster und zweiter Flüssigkeit, wobei diese nicht ineinander löslich sind, homogener, d. h. einheitlicher wird. Aufgrund der Homogenisierung, insbesondere einer Hochdruckhomogenisierung, wird eine optimierte Matrixflüssigkeit zur konvektiven Trocknung, insbesondere im Fluidisierungsapparat, erzeugt.

Weiterhin ist bei einer bevorzugten Fortbildung der Vorrichtung zwischen dem Behältersystem und dem Trocknungsapparat eine Granulationseinheit, bevorzugt ein Nassmischer, besonders bevorzugt ein High Shear Granulator, ein Vertikalgranulator, ein Rotorscheibengranulator oder dergleichen, zum Granulieren der Matrixflüssigkeit angeordnet. Nach einer diesbezüglichen Fortbildung der Vorrichtung ist nach der Granulationseinheit ein Extruder zum Extrudieren des Granulats angeordnet.

Bevorzugt sind der Homogenisator und/oder die Gratulationseinheit und/oder der Extruder fluidisch miteinander verbunden.

Entsprechend einer zusätzlichen vorteilhaften Ausgestaltung der Vorrichtung sind der Einlass und der Matrixflüssigkeitsauslass als eine Behältersystemöffnung ausgebildet. Der Vorteil einer derartigen Ausbildung von Einlass und Matrixflüssigkeitsauslass besteht in der konstruktiv einfachen Lösung sowie in einer einfacheren Abdichtung.

Die Vorrichtung wird verwendet zur Durchführung eines Verfahrens, wobei die Matrixflüssigkeit konvektiv getrocknet wird. Bevorzugt wird die Matrixflüssigkeit sprühgetrocknet oder in einem Fluidisierungsapparat durch eine Sprühgranulation, Sprühagglomeration oder Sprühverkapselung konvektiv zu einem Cannabinoid-Granulat getrocknet. Überraschenderweise wurde herausgefunden, dass sich bei einer konvektiven Trocknung der das Cannabinoid enthaltenden Matrixflüssigkeit in einem Fluidisierungsapparat, insbesondere einem Strahl- oder Wirbelschichtapparat, durch eine Sprühgranulation, Sprühagglomeration oder Sprühverkapselung nach erneuter Auflösung des Cannabinoid-Granulats im wässrigen Milieu eine stabile Emulsion aus erster und zweiter Flüssigkeit bildet, wobei eine Resorbierbarkeit der Cannabinoide deutlich verbessert ist und damit eine optimalere Bioverfügbarkeit gewährleistet wird. Die verbesserte Resorbierbarkeit und optimalere Bioverfügbarkeit der Cannabinoide resultiert aus homogeneren und kleineren Emulsionspartikeln (Öl-Tröpfchen in Wasser) nach dem erneuten Auflösen der Cannabinoid-Granulate im wässrigen Milieu im Vergleich zu den in der Matrixflüssigkeit enthaltenen Emulsionspartikeln (Öl-Tröpfchen in Wasser) vor der konvektiven Trocknung im Fluidisierungsapparat. Auch ist das Auflösungsverhalten der Granulate im wässrigen Milieu verbessert im Vergleich zu herkömmlichen Produkten, wie einem Pressling. Zudem wurde überraschend eine Reduzierung des mittleren Durchmessers x sowie der Äquivalenzdurchmesser nach der konvektiven Trocknung im Fluidisierungsapparat festgestellt. Die Cannabinoid-Granulate können sehr gut in Stickpacks abgefüllt oder zu Tabletten verpresst oder in Kapseln weiterverarbeitet werden.

Als Cannabinoide werden Transformationsprodukte und synthetische Analoga einiger Terpenphenole bezeichnet. Auch körpereigene Substanzen, die ähnliche pharmakologische Eigenschaften wie Cannabinoide aufweisen, sogenannte Endocannabinoide, und Stoffe anderer Pflanzen als der Hanfpflanze, die eine ähnliche oder gleiche Wirkung wie die Cannabinoide aufweisen, sogenannte Phytocannabinoide, werden vorliegend als Cannabinoide bezeichnet. Beispielsweise kann das Cannabinoid eines oder mehrere von Cannabidiol (CBD), Cannabinol (CBN) und Δ9-Tetrahydrocannabinol (THC) oder anderen beinhalten.

Entsprechend einer diesbezüglichen Fortbildung des Verfahrens wird zuerst ein Cannabinoid in der ersten Flüssigkeit gelöst danach die erste Flüssigkeit mit der zweiten Flüssigkeit zur Bildung der Emulsion vermischt.

Zudem wird bei einer diesbezüglichen Ausgestaltung des Verfahrens zuerst die erste Flüssigkeit mit der zweiten Flüssigkeit zur Bildung der Emulsion vermischt und danach ein Cannabinoid in der Emulsion gelöst.

Bevorzugt besteht die erste Flüssigkeit aus der Gruppe der Lipide, Alkohole, Öle und/oder einer beliebigen Mischung daraus. Die Auswahl der ersten Flüssigkeit ist von großer Bedeutung beispielsweise für die Resorbierbarkeit von Cannabinoiden im Magen-Darm-Trakt des Menschen. Gleichzeitig ist die Auswahl der ersten Flüssigkeit auch hinsichtlich einer eventuellen Geschmacksmaskierung bedeutsam. Hier kommen insbesondere Öle, wie bspw. Orangenöl in Frage.

Weiter bevorzugt ist die zweite Flüssigkeit eine wässrige Lösung oder Wasser.

In einer sehr vorteilhaften Ausgestaltung des Verfahrens ist die erste Flüssigkeit ein Öl bzw. eine Mischung verschiedener Öle, insbesondere ein Raps-, Orangen- oder Kokosöl, und/oder ein Alkohol bzw. eine Mischung verschiedener Alkohole, insbesondere Ethanol, bevorzugt mit einer Reinheit von > 80 %, und die zweite Flüssigkeit Wasser und somit eine Öl-in-Wasser-Emulsion oder eine alkoholische Lösung.

Als Emulgatoren werden Hilfsstoffe zur Herstellung und Stabilisierung von Emulsionen bezeichnet. Hierbei handelt es sich bevorzugt um grenzflächenaktive Stoffe, die dazu dienen zwei nicht miteinander mischbare Flüssigkeiten, wie zum Beispiel Öl und Wasser, zu einem fein verteilten Gemisch, der sogenannten Emulsion, zu vermengen und diese zu stabilisieren.

Gemäß einer vorteilhaften Weiterbildung des Verfahrens wird der zweiten Flüssigkeit vor dem Vermischen mit der ersten Flüssigkeit ein Emulgator zugegeben.

Nach einer weiteren zum Vorteil gereichenden Weiterbildung des Verfahrens wird der Emulsion vor dem konvektiven Trocknen ein Emulgator zugegeben.

Zudem wird der Matrixflüssigkeit bevorzugt vor dem konvektiven Trocknen ein Emulgator zugegeben.

Die Zugabe eines Emulgators oder auch mehrerer Emulgatoren hat den Vorteil, dass sich die erste und die zweite Flüssigkeit in der Emulsion feiner verteilen, wodurch sich auch eine für die konvektive Trocknung einem konvektiven Trocknungsapparat, bspw. einem Trommeltrockner, einem Vakuumtrockner oder insbesondere in einem Fluidisierungsapparat, vorzugsweise einem Strahl- oder Wirbelschichtapparat, besser geeignete Matrixflüssigkeit herstellen lässt. Die Zugabe des einen oder der mehreren Emulgatoren, wie bspw. Hi-Cap 100 (modifizierte Stärke) oder dergleichen, erfolgt bevorzugt unter Rühren, vorzugsweise mittels einer Rühreinrichtung oder dergleichen, und/oder Temperatureinwirkung, d. h. der Zuführung oder Abfuhr von Wärme.

Des Weiteren wird nach einer zusätzlichen vorteilhaften Ausgestaltung des Verfahrens die Matrixflüssigkeit vor dem konvektiven Trocknen homogenisiert. Bevorzugt wird die Emulsion hochdruckhomogenisiert, besonders bevorzugt bei Drücken zwischen 50 bar und 250 bar, ganz besonders bevorzugt bei Drücken zwischen 100 bar und 200 bar, am meisten bevorzugt bei Drücken zwischen 125 bar und 175 bar. Durch die Homogenisierung wird der mittlere Durchmesser der in der Emulsion vorhandenen Tröpfchen der ersten Flüssigkeit stark reduziert, sodass das Gemisch aus erster und zweiter Flüssigkeit, wobei diese nicht ineinander löslich sind, homogener, d. h. einheitlicher wird. Aufgrund der Homogenisierung, insbesondere einer Hochdruckhomogenisierung, wird eine optimierte Matrixflüssigkeit zur konvektiven Trocknung, insbesondere im Fluidisierungsapparat, erzeugt.

Vorteilhafterweise wird die Matrixflüssigkeit vor der konvektiven Trocknung, insbesondere in einem Fluidisierungsapparat, auf einen oder mehrere Trägerstoffe aufgebracht. Durch Aufbringen der Matrixflüssigkeit auf einen oder mehrere Trägerstoffe vor der konvektiven Trocknung wird bevorzugt ein das Cannabinoid enthaltende Nassgranulat bei gleichzeitiger Verdichtung der Trägerstoffe erzeugt. Vorzugsweise wird die Matrixflüssigkeit nach dem Aufbringen auf einen oder mehrere Trägerstoffe extrudiert oder die Matrixflüssigkeit wird nach dem Aufbringen auf einen oder mehrere Trägerstoffe einer pelletiert.

Nach einer zusätzlichen vorteilhaften Ausgestaltung des Verfahrens werden in dem Fluidisierungsapparat Trägerpartikel vorgelegt. Die Trägerpartikel, insbesondere bspw. Maltrodextrin, Mannitol, Cellulosen, Lactosen, Xylitol oder eine Mischung daraus, dienen als Keime für den Aufbau der Cannabinoid-Granulate. Teilweise verdunstet die Matrixflüssigkeit und es entstehen sprühgetrocknete Granulatkeime aus der Matrixflüssigkeit selbst.

Vorteilhafterweise werden der Lösung, Emulsion oder Matrixflüssigkeit Additive vor der konvektiven Trocknung zugegeben. Durch die Zugabe von Additiven, wie insbesondere Kohlenhydrate, bspw. Maltrodextrin, Stärke, Zucker, bspw. Fructose oder Saccharose, Salze, wie Magnesiumstearat, Aromen oder dergleichen, kann eine Geruchs-, Geschmacksmaskierung der Cannabinoid-Granulate erreicht werden. Additive können zudem Anwendung finden, wenn beispielsweise die Verpressbarkeit der Cannabinoid-Granulate bei der Herstellung von Tabletten angepasst werden muss oder das Freisetzungsprofil der Cannabinoide angepasst werden soll.

Entsprechend einer bevorzugten Weiterbildung des Verfahrens wird das Verfahren chargenweise oder kontinuierlich durchgeführt. Durch Variation der Anlagen- und Prozessparameter sind die gewünschten Endprodukteigenschaften wie Partikelgrößenverteilung, Auflösungsgeschwindigkeit, Schüttdichte oder Restfeuchte individuell und optimal einstellbar.

Nachfolgend wird die Erfindung anhand der beiliegenden Zeichnung näher erläutert dieser zeigen
- Figur 1: schematische Darstellung eines ersten Ausführungsbeispiels einer bevorzugten Vorrichtung zur Herstellung eines im Wesentlichen im wässrigen Milieu lösbaren Cannabinoid-Granulats,
- Figur 2: schematische Darstellung eines zweiten Ausführungsbeispiels einer bevorzugten Vorrichtung zur Herstellung eines im Wesentlichen im wässrigen Milieu lösbaren Cannabinoid-Granulats,
- Figur 3: eine Summenverteilungskurve und eine Dichteverteilungskurve Öl-Tröpfchen einer Öl-in-Wasser Emulsion (mit Rapsöl) vor der Trocknung im Strahlschichtapparat,
- Figur 4: eine Summenverteilungskurve und eine Dichteverteilungskurve Öl-Tröpfchen einer Öl-in-Wasser Emulsion (mit Rapsöl) nach Auflösung der im Strahlschichtapparat getrockneten Cannabinoid-Granulate in Wasser,
- Figur 5: eine Summenverteilungskurve und eine Dichteverteilungskurve Öl-Tröpfchen einer Öl-in-Wasser Emulsion (mit Kokosöl) vor der Trocknung im Strahlschichtapparat und
- Figur 6: eine Summenverteilungskurve und eine Dichteverteilungskurve Öl-Tröpfchen einer Öl-in-Wasser Emulsion (mit Kokosöl) nach Auflösung der im Strahlschichtapparat getrockneten Cannabinoid-Granulate in Wasser.

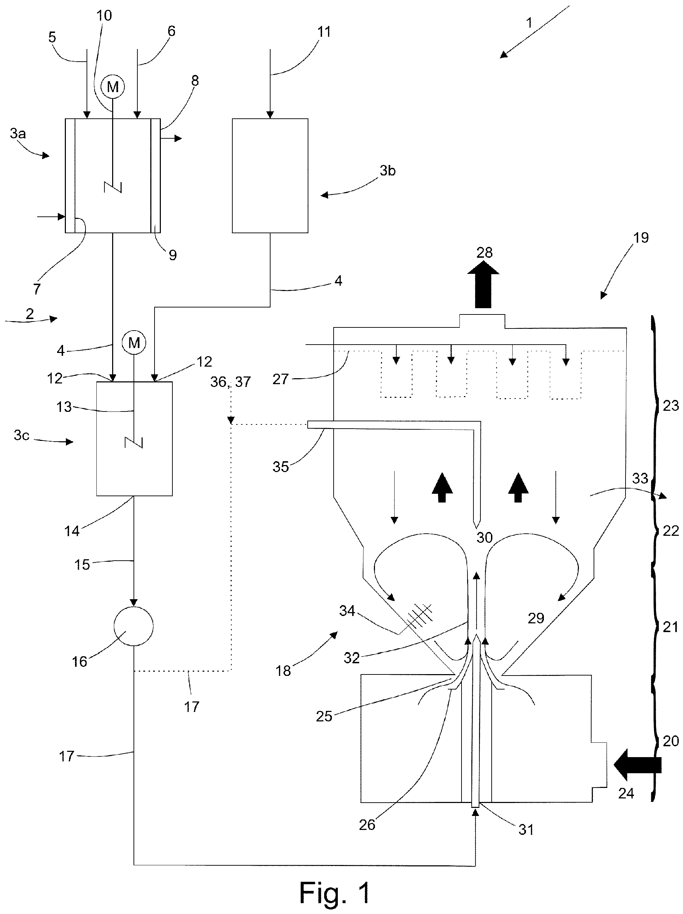

Fig. 1 zeigt eine schematische Darstellung eines ersten Ausführungsbeispiels einer bevorzugten Vorrichtung 1 zur Herstellung eines im Wesentlichen im wässrigen Milieu lösbaren Cannabinoid-Granulats.

Die Vorrichtung 1 umfasst ein Behältersystem 2 zur Herstellung der Matrixflüssigkeit. Im Ausführungsbeispiel gemäß Fig. 1 weist das Behältersystem 2 drei Behälter 3 auf, wobei diese untereinander fluidisch verbunden sind. Die fluidische Verbindung der Behälter 3 wird hierbei mittels Rohrleitungen 4 realisiert.

Der zum Beheizen oder Kühlen doppelwandig ausgebildete erste Behälter 3a des Behältersystems 2 verfügt über einen Cannabinoid-Einlass 5 und einen Flüssigkeit-Einlass 6 für eine aus der Gruppe der Lipide, Alkohole, Öle und/oder einer beliebigen Mischung daraus, besonders bevorzugt jedoch aus einem Raps-, Orangen- oder Kokosöl bestehenden ersten Flüssigkeit.

Durch den zwischen Behälterinnenwand 7 und Behälteraußenwand 8 aufgespannten Behältermantelraum 9 ist der erste Behälter 3a mittels eines durch den Behältermantelraum 9 strömenden Heizmediums temperierbar. Vorzugsweise wird der Behälter 3a beheizt, um ein in der ersten Flüssigkeit ggf. nicht gut lösbares Cannabinoid besser aufzulösen. Die Temperiereinrichtung ist nicht zwingend notwendig.

Darüber hinaus verfügt der erste Behälter 3a über eine motorbetriebene Rühreinrichtung 10 zum Rühren der ersten Flüssigkeit. Die Rühreinrichtung ist nicht zwingend erforderlich.

Für eine zweite Flüssigkeit, insbesondere eine wässrige Lösung oder Wasser, umfasst das Behältersystem 2 einen einen Flüssigkeit-Einlass 11 aufweisenden zweiten Behälter 3b.

Der erste und zweite Behälter 3a, 3b sind jeweils über eine Rohrleitung 4 mit Einlässen 12 eines dritten Behälters 3c verbunden. Die das Cannabinoid enthaltende erste Flüssigkeit und die zweite Flüssigkeit werden über Rohrleitungen 4 in den dritten Behälter 3c gefördert, bspw. mittels Pumpen.

Im dritten Behälter 3c wird durch Mischen der das Cannabinoid enthaltenden ersten Flüssigkeit und der zweiten Flüssigkeit, wobei die erste und die zweite Flüssigkeit miteinander eine Emulsion bilden, die Matrixflüssigkeit hergestellt. Bevorzugt wird diese unter Rühren mittels einer motorbetriebenen Rühreinrichtung 13 im dritten Behälter 3c erzeugt. Die hergestellte Matrixflüssigkeit verlässt das Behältersystem 2 zur Herstellung der Matrixflüssigkeit über einen Matrixflüssigkeitsauslass 14.

Die Matrixflüssigkeit ist auch durch andere Teilschritte herstellbar. Bspw. durch ein einfaches Zusammenmischen aller Komponenten in einem einzigen Behälter.

Die Matrixflüssigkeit wird über eine Rohrleitung 15 in einen Homogenisator 16 zum Homogenisieren der Matrixflüssigkeit, bevorzugt einen Hochdruckhomogenisator, gefördert, bspw. mittels einer Pumpe. Die homogenisierte Matrixflüssigkeit wird anschließend vom Homogenisator 16 über die Rohrleitung 17 in den Fluidisierungsapparat 18 gefördert, bspw. mittels einer Pumpe.

In dem Ausführungsbeispiel der Fig. 1 ist der Trocknungsapparat als Fluidisierungsapparat 18 zur Herstellung eines im Wesentlichen im wässrigen Milieu lösbaren Cannabinoid-Granulats ausgebildet, wobei der Fluidisierungsapparat 18 hier als Strahlschichtapparat 19 ausgebildet ist. Der Aufbau des Strahlschichtapparates 19 umfasst von unten nach oben eine Verteilerkammer 20, einen Prozessraum 21, eine Expansionszone 22 und ein Abluftteil 23.

Das zur Trocknung der herzustellenden Cannabinoid-Granulate erforderliche Prozessgas 24 wird der Verteilerkammer 20 zugeführt, wo sich das Prozessgas 24 verteilt und über eine Spaltöffnung 25 und ein Prozessgasumlenkteil 26 in einen Prozessraum 21 vorzugsweise als eine Art Freistrahl eintritt.

Im Weiteren kann sich der Apparatequerschnitt optional in der Expansionszone 22 vergrößern, sodass sich die Geschwindigkeit der Prozessgasströmung nach oben hin stetig verringert. Das Prozessgas 24 verlässt den Strahlschichtapparat 19 bevorzugt als durch ein Entstaubungssystem 27, insbesondere Filterpatronen oder Textilfilterelemente, aufgereinigtes Abgas 28.

Im Prozessraum 21 befinden sich als Startermaterial bezeichnete Trägerpartikel, wie bspw. Mannitol, Cellulose, Xylitol oder dergleichen, die durch das Prozessgas 24 nach oben in Richtung des Entstaubungssystems 27 mitgerissen werden. Im oberen Bereich des Prozessraumes 21 sowie in der darüber befindlichen Expansionszone 22 nimmt die Prozessgasgeschwindigkeit ab, sodass die aufwärtsströmenden Trägerpartikel seitlich aus dem Prozessgasstrom heraustreten und in den Prozessraum 21 zurückfallen. Der Prozessraum 21 wird im unteren Bereich von geneigten Seitenflächen 28 begrenzt. Aufgrund der geneigten Seitenflächen 28 werden die Trägerpartikel unter Wirkung der Schwerkraft über die Rücklaufzone 29 in Richtung der Spaltöffnung 25 befördert, wo sie anschließend wieder vom Prozessgas 24 in den Prozessraum 21 mitgerissen werden.

Durch diesen Mechanismus bildet sich eine sehr gleichförmige Feststoffzirkulation 30 der Trägerpartikel aus. Im unteren Bereich des Prozessraums 21 werden eine oder mehrere Sprüheinrichtungen 31, vorzugsweise eine Sprühdüse oder dergleichen, angeordnet die gleichgerichtet zum Prozessgas 24 nach oben sprühen und zum Einbringen der Matrixflüssigkeit dienen. Ein solches Einbringen der Matrixflüssigkeit im unteren Bereich des Prozessraums 21 wird als Bottom-Spray bezeichnet.

Die Düse, bevorzugt eine Zweistoffdüse, ist konfiguriert, um Tröpfchen mit einer Tröpfchengröße von 1 *µ*m bis 200 *µ*m, bevorzugt von 10 *µ*m bis 100 *µ*m, besonders bevorzugt zwischen 20µm und 60 *µ*m zu versprühen. Ganz besonders bevorzugt haben die versprühten Tröpfchen eine Tröpfchengröße von 25*µ*m bis 40*µ*m, am meisten bevorzugt von 30*µ*m. Durch die Einstellung der Tröpfchengröße mittels der Düse und der die Düse beaufschlagten Druckluft sind die beim Versprühen auftretenden Scherkräfte genau einstellbar, sodass eine sehr homogene Tröpfchengröße der zu versprühenden Emulsion oder Lösung erziel- bzw. erreichbar ist. Die Tröpfchen lagern sich an den Trägerpartikeln, wie Cellulose, Lactose oder Xylitol, an und es findet bevorzugt eine Filmverdampfung statt.

Aufgrund der sehr vorteilhaften Wärme- und Stoffübertragung sowie der hohen Trägerpartikelzirkulation im Bedüsungsbereich 32 des Prozessraums 21 des Strahlschichtapparates 19 wird erreicht, dass sich die Matrixflüssigkeit weitestgehend an den Trägerpartikeln abscheidet und diese somit gleichmäßig an den Partikeloberflächen benetzt werden. Das gleichmäßige Benetzen bei einer gleichzeitig hohen Partikelzirkulation zwischen Bedüsungsbereich 32 und Rücklaufzone 29 bewirkt, dass ein sehr gleichmäßiger Flüssigkeitsfilm auf den Trägerpartikeln gebildet wird. Durch den Trocknungsprozess verdampft die Matrixflüssigkeit und verlässt mit dem Abgas 28 den Strahlschichtapparat 19. Das in der Matrixflüssigkeit enthaltene Cannabinoid verbleibt auf der Partikeloberfläche der Trägerpartikel, sodass die entstehenden Cannabinoid-Granulate sehr gleichförmig und homogen wachsen.

Der Austrag 33 der Cannabinoid-Granulate kann beispielsweise durch einen Überlauf oder durch ein volumetrisches Austragsorgan, insbesondere eine Zellenradschleuse, oder auch durch einen Schwerkraftsichter, vorzugsweise einen mit Sichtgas beaufschlagte Zick-Zack-Sichter oder einen Steigrohrsichter, realisiert sein.

Mechanische Aggregate 34, wie bspw. Zerkleinerer, Häcksler usw., können nach Bedarf im Prozessraum 21, bevorzugt in der der Rücklaufzone 29, angeordnet sein, um durch Zerkleinerung ausreichend feine Partikel als Granulatkeime für den Granulatbildungsprozess zu erzeugen.

Im Prozessraum 21 oder in den darüber liegenden Apparateteilen, der Expansionszone 22 und dem Abluftteil 23, können optional eine oder mehrere Sprüheinrichtungen 35 angeordnet sein, die vorzugsweise nach unten sprühen. Über die Sprüheinrichtung 35 kann ebenfalls die flüssige Matrixflüssigkeit in den Prozessraum 21 des Strahlschichtapparates 19 eingedüst werden. Alternativ können über einige der Sprüheinrichtungen 31, 35 Additive 36 oder andere Komponenten 37 in flüssiger Form eingesprüht und somit in die Granulatstruktur homogen eingebettet werden.

In der bevorzugten Vorrichtung 1 kann zwischen dem Behältersystem 2 und dem als Strahlschichtapparat 19 ausgebildeten Fluidisierungsapparat 18 eine nicht gezeigte Granulationseinheit zum Granulieren der Matrixflüssigkeit angeordnet sein. Zudem besteht die Möglichkeit nach der Granulationseinheit ein Extruder zum Extrudieren des Granulats anzuordnen. Bevorzugt sind der Homogenisator 16 und/oder die Gratulationseinheit und/oder der Extruder fluidisch miteinander verbunden.

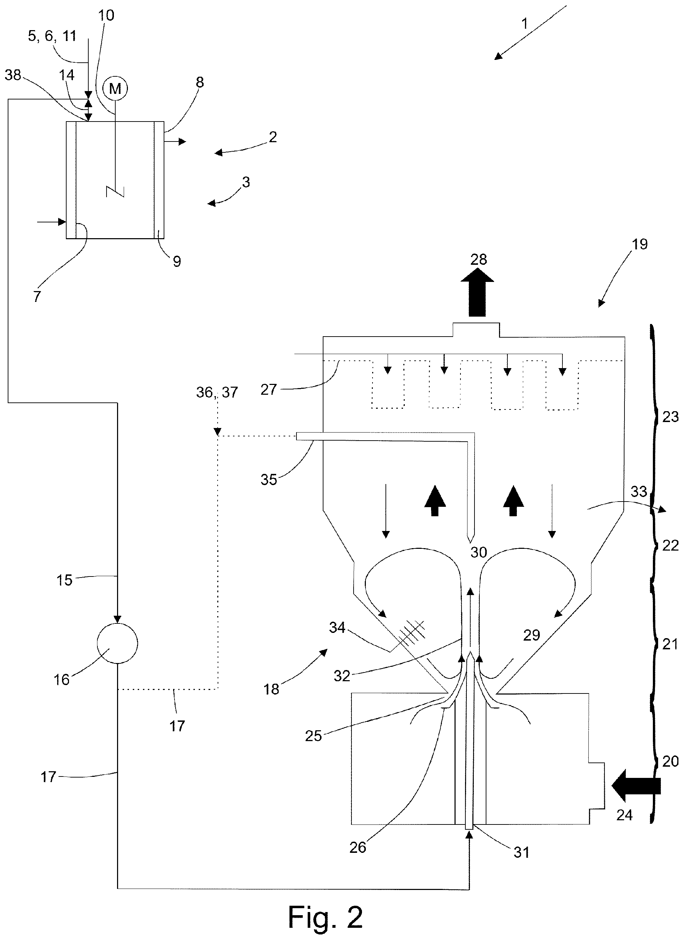

In der Fig. 2 wird eine schematische Darstellung eines zweiten Ausführungsbeispiels einer bevorzugten Vorrichtung 1 zur Herstellung eines im Wesentlichen im wässrigen Milieu lösbaren Cannabinoid-Granulats gezeigt.

Die Vorrichtung 1 umfasst ein Behältersystem 2 zur Herstellung der Matrixflüssigkeit. Im zweiten Ausführungsbeispiel gemäß Fig. 2 weist das Behältersystem 2 einen einzigen Behälter 3 auf.

Der zum Beheizen oder Kühlen doppelwandig ausgebildete Behälter 3 des Behältersystems 2 verfügt über einen Cannabinoid-Einlass 5 und einen Flüssigkeit-Einlass 6 für eine aus der Gruppe der Lipide, Alkohole, Öle und/oder einer beliebigen Mischung daraus, besonders bevorzugt jedoch aus einem Raps-, Orangen- oder Kokosöl bestehenden ersten Flüssigkeit sowie einen Flüssigkeit-Einlass 11 für eine zweite Flüssigkeit, insbesondere eine wässrige Lösung oder Wasser.

Durch den zwischen Behälterinnenwand 7 und Behälteraußenwand 8 aufgespannten Behältermantelraum 9 ist der erste Behälter 3a mittels eines durch den Behältermantelraum 9 strömenden Heizmediums temperierbar. Vorzugsweise wird der Behälter 3a beheizt, um ein in der ersten Flüssigkeit ggf. nicht gut lösbares Cannabinoid besser aufzulösen. Die Temperiereinrichtung ist nicht zwingend notwendig.

Darüber hinaus verfügt der erste Behälter 3a über eine motorbetriebene Rühreinrichtung 10 zum Rühren der ersten Flüssigkeit. Die Rühreinrichtung ist nicht zwingend erforderlich.

Im Behälter 3 wird durch Mischen der das Cannabinoid enthaltenden ersten Flüssigkeit und der zweiten Flüssigkeit, wobei die erste und die zweite Flüssigkeit miteinander eine Emulsion bilden, die Matrixflüssigkeit hergestellt. Bevorzugt wird diese unter Rühren mittels einer motorbetriebenen Rühreinrichtung 13 im Behälter 3 erzeugt. Die hergestellte Matrixflüssigkeit verlässt das Behältersystem 2 zur Herstellung der Matrixflüssigkeit über einen Matrixflüssigkeitsauslass 14. Der Einlass 5, 6, 11 und der Matrixflüssigkeitsauslass 14 sind im Ausführungsbeispiel als eine Behältersystemöffnung 38 ausgebildet.

Die Matrixflüssigkeit wird über eine Rohrleitung 15 in einen Homogenisator 16 zum Homogenisieren der Matrixflüssigkeit, bevorzugt einen Hochdruckhomogenisator, gefördert, bspw. mittels einer Pumpe. Die homogenisierte Matrixflüssigkeit wird anschließend vom Homogenisator 16 über die Rohrleitung 17 in den Fluidisierungsapparat 18 gefördert, bspw. mittels einer Pumpe.

Der weitere konvektive Trocknungsprozess mittels Fluidisierungsapparat erfolgt entsprechend dem ersten in Fig. 1 beschrieben Ausführungsbeispiel.

Im Nachgang können die erzeugten Cannabinoid-Granulate bspw. in einem Trommelcoater beschichtet werden. Dies hat den Vorteil, dass die Cannabinoid-Granulate zum Beispiel ein Magensaftresistente Beschichtung oder eine Geschmacksmaskierung erhalten können.

Nachfolgend werden Beispiele für die Herstellung einer Matrixflüssigkeit sowie deren Weiterverarbeitung im Fluidisierungsapparat aufgezeigt.

Beispiel 1: Die Herstellung der Cannabinoid-Granulate im als Strahlschichtapparat ausgebildeten Fluidisierungsapparat erfolgte im Chargen- bzw. Batchbetrieb als Bottom-Spray.

Als Startmaterial im Fluidisierungsapparat wurden 500 g Mannitol verwendet.

Die Matrixflüssigkeit weist 200 g Hi-Cap 100, 200 g Maltrodextrin, 1365 g Wasser, 5 g Cannabidiol (CBD) und 50 g Rapsöl. Hierbei wurde das Cannabidiol (CBD) in Rapsöl als erste Flüssigkeit und Maltrodextrin und Hi-Cap 100 in Wasser als zweite Flüssigkeit gelöst. Anschließend wurden das CBD enthaltende Rapsöl und das Maltrodextrin und Hi-Cap 100 enthaltende Wasser miteinander unter Bildung einer Emulsion vermischt und die so Matrixflüssigkeit hergestellt. Maltrodextrin und Hi-Cap 100 dienen hierbei als Additive zur Ausbildung einer Matrixstruktur in der Matrixflüssigkeit.

Die Sprühraten wurden zwischen 10 bis 15 g/min gewählt. Das hergestellte Cannabinoid-Granulat löste sich gut in Wasser auf und bildete dort eine stabile Öl-in-Wasser Emulsion. Die Restfeuchte des Cannabinoid-Granulats betrug 1,8 %.

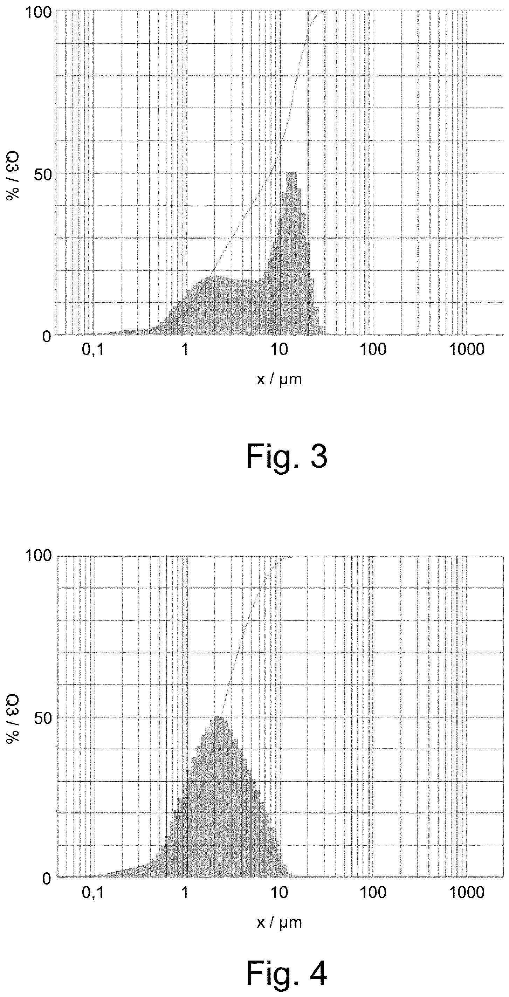

Die Matrixflüssigkeit ist im Anschluss an deren Herstellung mit einem Laserbeugungs-System Cilas 1190 LD (Quantachrome) analysiert worden. Die Korngrößen der in der Emulsion der Matrixflüssigkeit vorhandenen CBD enthaltenen Öl-Tröpfchen wird in Fig. 3 dargestellt. Die Summenverteilungskurve weist mit Äquivalentdurchmessern x₁₀ = 1,17 µm, x₅₀ = 7,97 µm und x₉₀ = 18,37 µm einen mittleren Durchmesser von x = 8,87 µm auf. Zudem zeigt die Dichteverteilungskurve mit ihren zwei Maxima eine bimodale Verteilung.

Nach Herstellung des Cannabinoid-Granulats und erneuter Auflösung des Cannabinoid-Granulats in Wasser hat sich im wässrigen Milieu eine stabile Öl-in-Wasser Emulsion gebildet.

Die im wässrigen Milieu gelösten Cannabinoid-Granulate wurden in Wasser aufgelöst und mit dem Laserbeugungs-System Cilas 1190 LD (Quantachrome) wurde erneut die Korngröße der CBDenthaltenden Öl-Tröpfchen analysiert. Fig. 4 zeigt die Analyse. Hierbei weist die Summenverteilungskurve mit Äquivalentdurchmessern x₁₀ = 0,82 *µ*m, x₅₀ = 2, 33 µm und x₉₀ = 6, 26 *µ*m einen mittleren Durchmesser von x = 3,01 *µ*m auf. Zudem zeigt die Dichteverteilungskurve eine monomodale Verteilung. Die Öl-Tröpfchen zeigen eine sehr homogene Dichteverteilung mit deutlich geringeren Durchmessern. Dies führt zur einer deutlich verbesserten Resorbierbarkeit von CBD und damit zu einer optimaleren Bioverfügbarkeit von CBD. Diese verbesserte Resorbierbarkeit der Cannabinoide resultiert aus homogeneren und kleineren Emulsionspartikeln.

Beispiel 2: Die Herstellung der Cannabinoid-Granulate im als Strahlschichtapparat ausgebildeten Fluidisierungsapparat erfolgte im Chargen- bzw. Batchbetrieb als Bottom-Spray.

Als Startmaterial im Fluidisierungsapparat wurden 250 g Mannitol und 250 g Maltrodextrin, d. h. im Verhältnis 1:1, verwendet.

Die Matrixflüssigkeit weist 200 g Hi-Cap 100, 200 g Maltrodextrin, 1417 g Wasser, 5,5 g Cannabidiol (CBD) und 51 g Kokosöl. Hierbei wurde das Cannabidiol (CBD) in Kokosöl als erste Flüssigkeit und Maltrodextrin und Hi-Cap 100 in Wasser als zweite Flüssigkeit gelöst. Anschließend wurden das CBD enthaltende Kokosöl und das Maltrodextrin und Hi-Cap 100 enthaltende Wasser miteinander unter Bildung einer Emulsion vermischt und die so Matrixflüssigkeit hergestellt. Maltrodextrin und Hi-Cap 100 dienen hierbei als Additive zur Ausbildung einer Matrixstruktur in der Matrixflüssigkeit.

Die Sprühraten wurden im Vergleich zum Beispiel 1 erhöht. Zudem wurde der Volumenstrom der Prozessluft verringert, um den Produktverlust an den Filtern zu minimieren. Das hergestellte Cannabinoid-Granulat löste sich gut in Wasser auf und bildete dort eine stabile Öl-in-Wasser Emulsion. Die Restfeuchte des Cannabinoid-Granulats betrug 3,4 %.

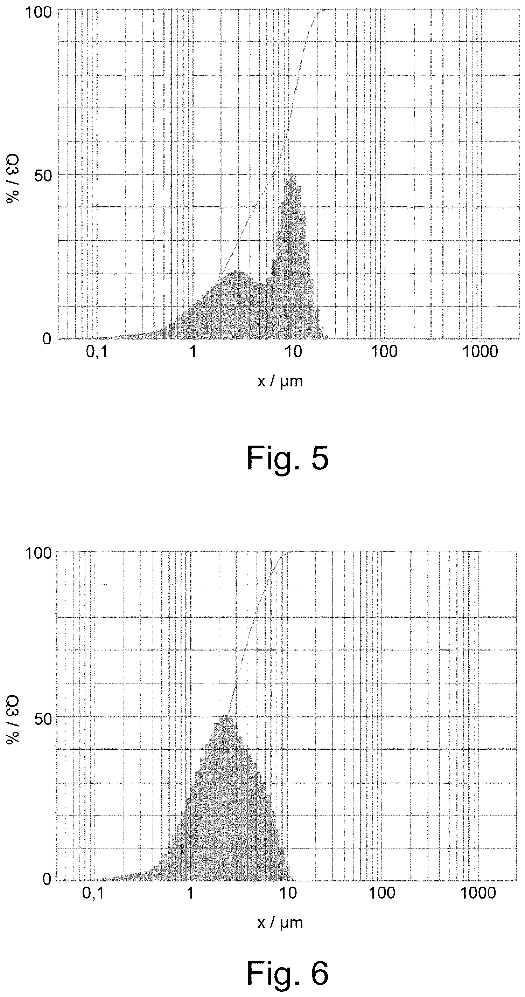

Die Matrixflüssigkeit ist im Anschluss an deren Herstellung mit einem Laserbeugungs-System Cilas 1190 LD (Quantachrome) analysiert worden. Die Korngrößen der in der Emulsion der Matrixflüssigkeit vorhandenen CBD enthaltenen Öl-Tröpfchen wird in Fig. 5 dargestellt. Die Summenverteilungskurve weist mit Äquivalentdurchmessern x₁₀ = 1,18 *µ*m, x₅₀ = 6, 99 *µ*m und x₉₀ = 15,17 *µ*m einen mittleren Durchmesser von x = 7,58 *µ*m auf.

Zudem zeigt die Dichteverteilungskurve mit ihren zwei Maxima eine bimodale Verteilung.

Nach Herstellung des Cannabinoid-Granulats und erneuter Auflösung des Cannabinoid-Granulats in Wasser hat sich im wässrigen Milieu eine stabile Öl-in-Wasser Emulsion gebildet.

Die im wässrigen Milieu gelösten Cannabinoid-Granulate wurden in Wasser aufgelöst und mit dem Laserbeugungs-System Cilas 1190 LD (Quantachrome) wurde erneut die Korngröße der CBDenthaltenden Öl-Tröpfchen analysiert. Fig. 6 zeigt die Analyse. Hierbei weist die Summenverteilungskurve mit Äquivalentdurchmessern x₁₀ = 0,90 *µ*m, x₅₀ = 2,48 *µ*m und x₉₀ = 6,24 *µ*m einen mittleren Durchmesser von x = 3,08 *µ*m auf. Zudem zeigt die Dichteverteilungskurve eine monomodale Verteilung. Die Öl-Tröpfchen zeigen eine sehr homogene Dichteverteilung mit deutlich geringeren Durchmessern. Dies führt zur einer deutlich verbesserten Resorbierbarkeit von CBD und damit zu einer optimaleren Bioverfügbarkeit von CBD. Diese verbesserte Resorbierbarkeit der Cannabinoide resultiert aus homogeneren und kleineren Emulsionspartikeln.

## Patentansprüche

1. Vorrichtung (1) zur Durchführung eines Verfahrens zur Herstellung eines im Wesentlichen im wässrigen Milieu lösbaren Cannabinoid-Granulats, wobei eine Matrixflüssigkeit aus einer ein Cannabinoid lösenden ersten Flüssigkeit oder aus einer ein Cannabinoid lösenden ersten Flüssigkeit und einer mit der ersten Flüssigkeit eine Emulsion bildenden zweiten Flüssigkeit und einem in der ersten Flüssigkeit oder der Emulsion gelösten Cannabinoid hergestellt wird, wobei die Matrixflüssigkeit in einem Fluidisierungsapparat (18) durch eine Sprühgranulation, Sprühagglomeration oder Sprühverkapselung konvektiv zu einem Cannabinoid-Granulat getrocknet wird, **dadurch gekennzeichnet, dass** die Vorrichtung (1) über ein mehrere untereinander über Rohrleitungen (4) zumindest teilweise fluidisch verbundene Behälter (3a, 3b, 3c) aufweisendes Behältersystem (2) zur Herstellung der Matrixflüssigkeit verfügt, das einen Cannabinoid-Einlass (5), einen Flüssigkeit-Einlass (6) für eine erste Flüssigkeit aus der Gruppe der Lipide, Alkohole, Öle und/oder einer beliebigen Mischung daraus, einen Flüssigkeit-Einlass (11) für eine zweite Flüssigkeit und einen Matrixflüssigkeitsauslass (14) aufweist, wobei die Vorrichtung einen ersten Behälter (3a) für die erste Flüssigkeit, einen zweiten Behälter (3b) für die zweite Flüssigkeit und einen einen Einlass (12) aufweisenden Behälter (3c) für die Emulsion aufweist, und wobei die Vorrichtung ferner einen mit dem Matrixflüssigkeitsauslass (14) des Behältersystems (2) fluidisch verbundenen konvektiven als Fluidisierungsapparat (18) ausgebildeten Trocknungsapparat aufweist.

2. Vorrichtung (1) nach Anspruch 1, **dadurch gekennzeichnet, dass** der Fluidisierungsapparat (18) als Strahlschichtapparat (19) oder als Wirbelschichtapparat ausgebildet ist.

3. Vorrichtung (1) nach Anspruch 1, **dadurch gekennzeichnet, dass** der Behälter (3c) für die Emulsion dem ersten oder zweiten Behälter (3a, 3b) entspricht.

4. Vorrichtung (1) nach einem der Ansprüche 1 bis 3, **dadurch gekennzeichnet, dass** der Fluidisierungsapparat (18) eine Düse zur Verdüsung der Emulsion oder Lösung aufweist.

5. Vorrichtung (1) nach Anspruch 4, **dadurch gekennzeichnet, dass** die Düse, bevorzugt eine Zweistoffdüse, konfiguriert ist, um Tröpfchen mit einer Tröpfchengröße von 1 *µ*m bis 200 *µ*m, bevorzugt von 10 *µ*m bis 100 *µ*m, besonders bevorzugt zwischen 20*µ*m und 60 *µ*m zu versprühen.

6. Vorrichtung (1) nach einem der Ansprüche 1 bis 5, **dadurch gekennzeichnet, dass** zwischen dem Behältersystem (2) und dem Fluidisierungsapparat (18) ein Homogenisator (16) zum Homogenisieren der Matrixflüssigkeit angeordnet ist.

7. Vorrichtung (1) nach einem der Ansprüche 1 bis 6, **dadurch gekennzeichnet, dass** zwischen dem Behältersystem (2) und dem Trocknungsapparat (18) eine Granulationseinheit, bevorzugt ein High Shear Granulator, ein Vertikalgranulator oder ein Rotorscheibengranulator, zum Granulieren der Matrixflüssigkeit angeordnet ist.

8. Vorrichtung (1) nach Anspruch 7, **dadurch gekennzeichnet, dass** nach der Granulationseinheit ein Extruder zum Extrudieren des Granulats angeordnet ist.

9. Vorrichtung (1) nach einem der Ansprüche 6 bis 8, **dadurch gekennzeichnet, dass** der Homogenisator (16) und/oder die Gratulationseinheit und/oder der Extruder fluidisch miteinander verbunden sind.

10. Vorrichtung (1) nach einem der Ansprüche 1 bis 9, **dadurch gekennzeichnet, dass** der Einlass (5, 6, 11, 12) und der Matrixflüssigkeitsauslass (14) als eine Behältersystemöffnung (38) ausgebildet sind.

## Claims

1. Apparatus (1) for carrying out a method for producing a cannabinoid granulate which is substantially soluble in an aqueous environment, wherein a matrix liquid is produced from a first liquid dissolving a cannabinoid or from a first liquid dissolving a cannabinoid and a second liquid forming an emulsion with the first liquid and a cannabinoid dissolved in the first liquid or the emulsion, wherein the matrix liquid is convectively dried to a cannabinoid granulate in a fluidization apparatus (18) by a spray granulation, spray agglomeration or spray encapsulation, **characterized in that** the apparatus (1) has a container system (2) for producing the matrix liquid, said container system having a plurality of containers (3a, 3b, 3c) which are at least partially fluidically connected to one another via pipelines (4), which [container system] has a cannabinoid inlet (5), a liquid inlet (6) for a first liquid from the group of lipids, alcohols, oils and/or any mixture thereof, a liquid inlet (11) for a second liquid and a matrix liquid outlet (14), wherein the apparatus has a first container (3a) for the first liquid, a second container (3b) for the second liquid and a container (3c) having an inlet (12) for the emulsion, and wherein the apparatus further has a convective drying apparatus which is fluidically connected to the matrix liquid outlet (14) of the container system (2) and is designed as a fluidization apparatus (18).

2. Apparatus (1) according to claim 1, **characterized in that** the fluidization apparatus (18) is designed as a spouted bed apparatus (19) or as a fluidized bed apparatus.

3. Apparatus (1) according to claim 1, **characterized in that** the container (3c) for the emulsion corresponds to the first or second container (3a, 3b).

4. Apparatus (1) according to one of claims 1 to 3, **characterized in that** the fluidization apparatus (18) has a nozzle for atomizing the emulsion or solution.

5. Apparatus (1) according to claim 4, **characterized in that** the nozzle, preferably a two-fluid nozzle, is configured to spray droplets with a droplet size of 1 *µ*m to 200 *µ*m, preferably of 10 *µ*m to 100 *µ*m, particularly preferably between 20 *µ*m and 60 *µ*m.

6. Apparatus (1) according to one of claims 1 to 5, **characterized in that** a homogenizer (16) for homogenizing the matrix liquid is arranged between the container system (2) and the fluidization apparatus (18).

7. Apparatus (1) according to one of claims 1 to 6, **characterized in that** a granulation unit, preferably a high shear granulator, a vertical granulator or a rotor disk granulator, for granulating the matrix liquid is arranged between the container system (2) and the drying apparatus (18).

8. Apparatus (1) according to claim 7, **characterized in that** an extruder for extruding the granulate is arranged after the granulation unit.

9. Apparatus (1) according to one of claims 6 to 8, **characterized in that** the homogenizer (16) and/or the granulation unit and/or the extruder are fluidically connected to one another.

10. Apparatus (1) according to one of claims 1 to 9, **characterized in that** the inlet (5, 6, 11, 12) and the matrix liquid outlet (14) are formed as one container system opening (38).

## Revendications

1. Dispositif (1) pour la mise en œuvre d'un procédé pour la production d'un granulé de cannabinoïde soluble sensiblement en milieu aqueux, dans lequel un liquide matriciel est produit à partir d'un premier liquide dissolvant un cannabinoïde ou à partir d'un premier liquide dissolvant un cannabinoïde et d'un deuxième liquide formant une émulsion avec le premier liquide et d'un cannabinoïde dissous dans le premier liquide ou l'émulsion, dans lequel le liquide matriciel est séché par convexion en un granulé de cannabinoïde dans un appareil de fluidisation (18) par une granulation par pulvérisation, agglomération par pulvérisation ou encapsulation par pulvérisation, **caractérisé en ce que** le dispositif (1) dispose d'un système de récipients (2) présentant plusieurs récipients (3a, 3b, 3c) reliés fluidiquement au moins en partie par l'intermédiaire de conduites (4) pour la production du liquide matriciel, qui présente une entrée de cannabinoïde (5), une entrée de liquide (6) pour un premier liquide du groupe des lipides, alcools, huiles et/ou d'un mélange quelconque de ceux-ci, une entrée de liquide (11) pour un deuxième liquide et une sortie de liquide matriciel (14), dans lequel le dispositif présente un premier récipient (3a) pour le premier liquide, un deuxième récipient (3b) pour le deuxième liquide et un récipient (3c) présentant une entrée (12) pour l'émulsion, et dans lequel le dispositif présente en outre un appareil de séchage par convexion réalisé sous la forme d'un appareil de fluidisation (18) relié fluidiquement à la sortie de liquide matriciel (14) du système de récipients (2).

2. Dispositif (1) selon la revendication 1, **caractérisé en ce que** l'appareil de fluidisation (18) est réalisé sous la forme d'un appareil à lit jaillissant (19) ou sous la forme d'un appareil à lit fluidisé.

3. Dispositif (1) selon la revendication 1, **caractérisé en ce que** le récipient (3c) pour l'émulsion correspond au premier ou deuxième récipient (3a, 3b).

4. Dispositif (1) selon l'une quelconque des revendications 1 à 3, **caractérisé en ce que** l'appareil de fluidisation (18) présente une buse pour l'atomisation de l'émulsion ou de la solution.

5. Dispositif (1) selon la revendication 4, **caractérisé en ce que** la buse, de préférence une buse à deux substances, est configurée pour pulvériser des gouttelettes avec une taille de gouttelette de 1 µm à 200 µm, de préférence de 10 µm à 100 µm, de manière particulièrement préférée comprise entre 20 µm et 60 µm.

6. Dispositif (1) selon l'une quelconque des revendications 1 à 5, **caractérisé en ce qu'**un homogénéisateur (16) pour homogénéiser le liquide matriciel est disposé entre le système de récipients (2) et l'appareil de fluidisation (18).

7. Dispositif (1) selon l'une quelconque des revendications 1 à 6, **caractérisé en ce qu'**une unité de granulation, de préférence un granulateur à cisaillement élevé, un granulateur vertical ou un granulateur à disques de rotor, pour granuler le liquide matriciel, est disposé entre le système de récipients (2) et l'appareil de séchage (18).

8. Dispositif (1) selon la revendication 7, **caractérisé en ce qu'**un extrudeur pour extruder le granulat est disposé après l'unité de granulation.

9. Dispositif (1) selon l'une quelconque des revendications 6 à 8, **caractérisé en ce que** l'homogénéisateur (16) et/ou l'unité de granulation et/ou l'extrudeur sont reliés fluidiquement les uns aux autres.

10. Dispositif (1) selon l'une quelconque des revendications 1 à 9, **caractérisé en ce que** l'entrée (5, 6, 11, 12) et la sortie de liquide matriciel (14) sont réalisées sous la forme d'une ouverture de système de récipients (38).
